# FASCICULE DE BREVET EUROPEEN

(11) **EP 0 564 328 B1**
(45) Date de publication et mention de la délivrance du brevet: **04.06.1997**
(21) Numéro de dépôt: 93400773.3
(22) Date de dépôt: 24.03.1993
(51) Int. Cl.: C10G 45/40, C07C 7/163, B01J 23/62, B01J 23/825

(54) **Procédé d'hydrogénation sélective des hydrocarbures**
Verfahren zur selektiven Hydrierung von Kohlenwasserstoffen
Process for the selective hydrogenation of hydrocarbons

(30) Priorité: 02.04.1992 FR 9204150
(43) Date de publication de la demande: 06.10.1993
(73) Titulaire: INSTITUT FRANCAIS DU PETROLE, 92502 Rueil-Malmaison (FR)
(72) Inventeur: Sarrazin, Patrick, F-92500 Rueil Malmaison (FR); Boitiaux, Jean-Paul, F-78300 Poissy (FR)

(56) Documents cités:
- DE-B- 1 004 316
- DE-B- 1 115 238
- FR-A- 2 103 122
- PATENT ABSTRACTS OF JAPAN 25 Juillet 1989
- PATENT ABSTRACTS OF JAPAN 18 Septembre 1981

## Description

De nombreux procédés de production d'oléfines, tels que le vapocraquage, le craquage catalytique et la viscoréduction produisent des coupes polluées par des molécules plus insaturées que les oléfines recherchées. La bonne utilisation de ces coupes pour la fabrication de produits finis implique l'élimination de ces molécules qui contiennent des doubles liaisons conjuguées et/ou des triples liaisons. L'hydrogénation sélective de celles ci en oléfines correspondantes est le procédé de choix pour s'en débarrasser tout en récupérant les oléfines recherchées.

Ces réactions d'hydrogénation sont généralement effectuées dans une gamme de températures comprises entre 20 et 200°C, sous une pression comprise entre 10 et 100 bar (1 et 10 Mégapascal) et avec une vitesse spatiale horaire comprise entre 1 et 40 m³/m³ de catalyseur/h. Les catalyseurs généralement utilisés sont constitués d'un ou plusieurs métaux déposés sur un support oxyde. Les métaux de base préférés sont couramment ceux du groupe VIII et plus particulièrement le nickel, le palladium et le platine. Les supports sont, quant à eux, souvent choisis parmi l'alumine, la silice, les silice-alumines, les aluminates ou encore le charbon.

La mise en oeuvre industrielle de tels catalyseurs se fait souvent en présence d'additifs visant à l'amélioration de la sélectivité de la réaction d'hydrogénation. Le composé le plus fréquemment employé étant le monoxyde de carbone comme revendiqué dans le brevet EP 0.081.041.

Le développement de catalyseurs plus performants du point de vue de l'activité et de la sélectivité a amené l'introduction d'autres métaux dans les formulations catalytiques. On peut citer, par exemple, l'argent (US-A-4409410 de la demanderesse) et l'or (US-A-4490481 de la demanderesse) qui améliorent très nettement les propriétés catalytiques des métaux du groupe VIII pour la réaction d'hydrogénation.
La demande de brevet JP-A-1110594 décrit un procédé d'hydrogénation sélective de dioléfines, en présence d'un catalyseur supporté au palladium, en présence de monoxyde de carbone (comme agent sélectivant) et en présence d'un donneur d'électron pourrait être choisi parmi les éthers, les alcools, les composés azotés, les hydrocarbures aromatiques, Na, K, Ag, Cu, Ga, In, Cr, Mo ou La.

La demande de brevet JP-A-56081306 décrit un procédé d'hydrogénation d'un polymère avec un catalyseur à base de palladium et d'un autre élément choisi parmi Au, B, Al, Ga, Ge, Sm, Si, Ge, Sb, Mo, Te, W et Re. Des copolymères butadiène/styrène ou butadiène/acrylonitrile sont aussi totalement hydrogénés pour leur partie butadiène sans que soit atteinte la partie styrène ou nitrile.

Il a été découvert dans la présente invention qu'il est possible de réaliser en l'absence de monoxyde de carbone l'hydrogénation de composés insaturés dioléfiniques et acétyléniques dans des charges contenant des monooléfines avec des sélectivités élevées en composés oléfiniques correspondants sans diminution de l'activité du métal de base (c'est-à-dire du groupe VIII), avec promotion et ceci sans additif dans le milieu réactionnel ni préparation d'un alliage bimétallique. On opère dans un réacteur continu ou discontinu en présence d'hydrogène sous une pression totale comprise entre 10 et 100 bar (1 et 10 Mégapascal) et de préférence entre 20 et 80 bar (2 et 8 Mégapascal), bien que l'on puisse opérer sans inconvénient, par exemple, jusqu'à 300 bar (30 Mégapascals), à une température comprise entre 0 et 200 degrés Celsius et de préférence entre 30 et 120 degrés Celsius en présence d'un nouveau catalyseur métallique. Ledit catalyseur renferme (a) au moins un métal du groupe VIII choisi parmi le nickel, le palladium, le platine, le rhodium et le ruthénium (le palladium, le platine et le nickel étant les métaux préférés) et dont le pourcentage pondéral est choisi entre 0,1 et 10 % et de préférence entre 0,2 et 5 % et (b) au moins un élément additionnel métallique choisi dans le groupe III.A constitué par le gallium et l'indium dont le pourcentage pondéral est choisi entre 0,01 et 10 % et de préférence entre 0,1 et 5 % et le rapport molaire élément métallique du groupe III sur métal du groupe VIII est avantageusement compris entre 0,2 et 5 et de préférence entre 0,3 et 2 (c) un support, choisi dans le groupe constitué par une silice, une alumine, une silice-alumine, un aluminate et un charbon. Avantageusement, les aluminates des éléments des groupes I.A, II.A ou II.B de la classification périodique peuvent être utilisés comme par exemple les aluminates de Ca, Mg, Ba, Zn, Na, K, Cd et les aluminates mixtes.

Le catalyseur peut être préparé par différentes procédures. Une procédure préférée est l'imprégnation du support, mais l'invention n'est pas limitée à une procédure déterminée. L'imprégnation consiste, par exemple, à mettre en contact le support préformé et une solution aqueuse ou organique d'un composé du métal ou des métaux choisi(s) dans le groupe III.A (gallium et l'indium) le volume de solution étant en excès par rapport au volume de rétention du support ou de préférence égal à ce volume. Après avoir laissé le contact entre le support et la solution pendant plusieurs heures, le support imprégné est filtré, lavé à l'eau distillé, séché et calciné sous air habituellement entre 110°C et 600°C et de préférence entre 110°C et 500°C. Avant dépôt du métal ou des métaux du groupe VIII, on peut avantageusement réduire le catalyseur sous hydrogène, on opère habituellement entre 50°C et 600°C et de préférence entre 90°C et 500°C ou à l'aide d'un réducteur organique en solution. Cette opération permet encore d'augmenter l'activité du catalyseur.

Le produit obtenu est alors imprégné par une solution organique (hydrocarbonée par exemple) ou aqueuse d'un métal du groupe VIII selon la nature du précurseur utilisé ; d'une manière particulièrement avantageuse, on utilise une solution de nitrate de palladium ou de nickel dans l'eau.

Le support ainsi imprégné est filtré, éventuellement lavé à l'eau distillée puis séché et calciné sous air habituellement entre environ 110°C et environ 600°C, et de préférence entre environ 110°C et environ 500°C, puis ensuite réduit sous hydrogène à une température habituellement comprise entre environ 50°C et environ 600°C et de préférence entre environ 80°C et environ 500°C. Les éléments des GVIII et GIII se trouvent alors sous forme d'oxyde et/ou métallique déposés sur le support.

Une autre méthode consiste à malaxer la poudre humide de support avec les précurseurs du catalyseur et à mettre ensuite en forme et sécher.

Les exemples des précurseurs métalliques utilisables dans la préparation du catalyseur sont les suivants:

Pour le métal du groupe VIII, on peut utiliser des composés tels que les chlorures, les nitrates, les composés halogèno-aminés, les composés aminés, les sels d'acides organiques solubles dans le solvant d'imprégnation.

On peut aussi utiliser des composés organométalliques d'un métal du groupe VIII en solution dans un solvant organique, par exemple un hydrocarbure. Comme exemple d'hydrocarbures on peut citer les hydrocarbures paraffiniques saturés dont la chaîne hydrocarbonée renferme de 6 à 12 atomes de carbone par molécule, les hydrocarbures naphténiques qui renferment de 6 à 12 atomes de carbone par molécule ou encore les hydrocarbures aromatiques renfermant de 6 à 12 atomes de carbone par molécule. A titre d'exemples de composés organométalliques de métal du groupe VIII on peut citer : les composés carbonyles, halogénocarbonyles et les acétylacétonates sans que cette liste soit limitative.

L'élément choisi dans le groupe constitué par le gallium et l'indium peut être introduit de préférence sous la forme d'au moins un composé inorganique choisi dans le groupe formé par les chlorures, les nitrates, les composés halogéno-aminés, les composés aminés et les sels d'acides organiques solubles dans les solvants d'imprégnation.

L'introduction du métal III.A est avantageusement effectuée à l'aide d'une solution aqueuse du composé inorganique du dit métal III.A.

L'élément choisi dans le groupe constitué par le gallium et l'indium peut aussi être introduit par l'intermédiaire de composés organométalliques en solution dans un solvant organique, par exemple un hydrocarbure. Comme exemple d'hydrocarbures on peut citer les hydrocarbures paraffiniques saturés dont la chaîne hydrocarbonée renferme de 6 à 12 atomes de carbone par molécule, les hydrocarbures naphténiques qui renferment de 6 à 12 atomes de carbone par molécule ou encore les hydrocarbures aromatiques renfermant de 6 à 12 atomes de carbone par molécule. A titre d'exemples de composés organométalliques de métal du groupe constitué par l'aluminium, le gallium et l'indium on peut citer : les alkyles, les alkoxydes, les acétates et les acétylacétonates sans que cette liste soit limitative.

Le support peut être de nature variée, comme déjà mentionné plus haut. Un support particulièrement adapté possède des caractéristiques spécifiques telles qu'une aire spécifique, déterminée par la méthode B.E.T., comprise entre 10 et 500 m² par gramme et de préférence comprise entre 50 et 500 m² par gramme et un volume poreux total de 0,2 à 1,3 cm³ par gramme de support.

Une fois les métaux fixés sur le support, le catalyseur subit avantageusement un traitement d'activation sous hydrogène à haute température, par exemple 50-600°C, afin d'obtenir une phase métallique active. La procédure de ce traitement sous hydrogène consiste par exemple en une montée lente de la température sous courant d'hydrogène jusqu'à la température maximale de réduction, comprise par exemple entre 50°C et 600°C et de préférence entre 80°C et 500°C, suivie d'un maintien pendant par exemple 1 à 6 heures à cette température.

Les exemples suivants, non limitatifs, illustrent l'invention.

### EXEMPLE 1 (comparatif)

On se propose dans cette exemple d'hydrogéner une charge constituée de 10 % poids de butadiène dans de l'heptane. La réaction est mise en oeuvre dans un réacteur discontinu parfaitement agité de type Grignard dans les conditions opératoires suivantes :
Pression = 20 bar
Température = 20°C

L'hydrogène utilisé est exempt de monoxyde de carbone.

Le catalyseur utilisé, appelé catalyseur A, est constitué de palladium à une teneur de 0,3 % poids déposé sur une alumine de transition de surface spécifique égale à 70 m²/g. Il est préparé par imprégnation à sec d'une alumine gamma tétragonale de volume poreux égal à 0,6 cm³/g à l'aide d'une solution de nitrate de palladium. Après imprégnation, l'échantillon est séché à une température de 120°C pendant 2 heures, puis calciné sous débit d'air à une température de 450°C pendant 2 heures. Avant test, on réduit le catalyseur sous débit d'hydrogène à une température de 150°C pendant 2 heures.

Au cours de l'avancement de la réaction des échantillons sont régulièrement prélevés et analysés par chromatographie en phase gazeuse de manière à suivre la transformation du butadiène en butènes et butane. Les résultats obtenus sont présentés dans le tableau suivant :

| **Temps (minutes)** | **Butadiène (% poids)** | **Butènes (% poids)** | **Butane (% poids)** |
|---|---|---|---|
| 0 | 100 | - | - |
| 1 | 80,85 | 19,1 | 0,05 |
| 2 | 75,75 | 24,15 | 0,1 |
| 3 | 66 | 33,85 | 0,15 |
| 4 | 54,5 | 45,33 | 0,17 |
| 5 | 43 | 56,8 | 0,2 |
| 6 | 32 | 67,7 | 0,21 |
| 7 | 20,5 | 79,28 | 0,22 |
| 8 | 9 | 90,7 | 0,3 |

### EXEMPLE 2 (selon l'invention)

Dans cet exemple on met en oeuvre la même réaction dans les mêmes conditions que dans l'exemple 1, mais cette fois on utilise successivement différents catalyseurs contenant 0,3 % poids de palladium et une teneur variable en gallium. Le support utilisé est identique à celui du catalyseur A monométallique de l'exemple 1. Différents lots de ce support sont imprégnés à sec par des solutions de nitrate de gallium de concentrations variables. Après imprégnation, les échantillons sont séchés à une température de 120°C pendant 2 heures, puis calcinés sous débit d'air à une température de 450°C pendant 2 heures. On procède alors au dépôt du palladium en utilisant la même méthode que celle décrite dans l'exemple 1 pour le catalyseur A. Avant test, on réduit les catalyseurs sous débit d'hydrogène à une température de 150°C pendant 2 heures.

Le tableau suivant présente la composition du produit après 8 minutes de réaction pour chacun des catalyseurs repérés par leur teneur en gallium ainsi que pour le catalyseur A monométallique de l'exemple 1.

| **Teneur en Ga (% poids)** | **Butadiène (% poids)** | **Butènes (%poids)** | **Butane (%poids)** |
|---|---|---|---|
| 0 | 9 | 90,7 | 0,3 |
| 0,07 | 6,57 | 93,16 | 0,27 |
| 0,21 | 5,55 | 94,2 | 0,25 |
| 0,42 | 7,5 | 92,22 | 0,28 |
| 0,82 | 8 | 91,7 | 0,3 |
| 1,19 | 10,3 | 89,4 | 0,3 |

On remarque que les échantillons ayant une teneur en gallium comprise entre 0,07 et 0,82 % poids présentent une activité supérieure à celle du catalyseur monométallique puisque au bout du même temps de réaction (8 minutes) la teneur en butadiène du produit est inférieure. De plus, on note que ces catalyseurs plus actifs que le monométallique sont aussi plus sélectifs vis à vis de l'hydrogénation consécutive des butènes. En effet, bien que la conversion du butadiène soit plus importante, on voit que la teneur en butènes est plus importante et que la formation de butane est diminuée.

### EXEMPLE 3 (selon l'invention)

Dans cet exemple on met en oeuvre la même réaction dans les mêmes conditions que dans l'exemple 1. On utilise cette fois un catalyseur B contenant 0,3 % poids de palladium et 0,24 % poids de gallium déposés sur le même support que dans l'exemple 1 suivant la même méthode de préparation que dans l'exemple 2. On utilise aussi un catalyseur C ayant la même composition mais qui diffère du catalyseur B par le fait que le précurseur au gallium sur alumine utilisé lors de la préparation a été réduit sous hydrogène à une température de 450°C pendant 2 heures avant le dépôt de palladium. La composition des produits obtenus après 8 minutes de réaction est présentée dans le tableau suivant :

| **Catalyseur** | **Butadiène (% poids)** | **Butènes (%poids)** | **Butane (%poids)** |
|---|---|---|---|
| B | 5,6 | 94,14 | 0,26 |
| C | 4,2 | 95,53 | 0,27 |

On voit que le catalyseur C est plus actif que le catalyseur B vis à vis de l'hydrogénation du butadiène. Un gain de sélectivité est aussi observé puisqu'avec une conversion en butadiène plus importante, la teneur en butènes est plus élevée pour le catalyseur C.

### EXEMPLE 4 (comparatif)

On se propose dans cette exemple d'hydrogéner une coupe C₃ de vapocraquage dont la composition est la suivante :
Propane = 3,59 %
Propylène = 92,14 %
Propyne (MA) = 1,78 %
Propadiène (PD) = 1,65 %

La réaction est mise en oeuvre en phase liquide dans un réacteur continu à lit fixe dans les conditions opératoires suivantes :
Pression = 24 bar
Température = 50°C
Vitesse spatiale horaire = 20 cm³ de charge/cm³ de catalyseur/h
Rapport molaire hydrogène sur propyne plus propadiène = 1,2

L'hydrogène utilisé est exempt de monoxyde de carbone. Le catalyseur utilisé est le catalyseur A de l'exemple 1.

Au cours du temps des échantillons de produit sont régulièrement prélevés et analysés par chromatographie en phase gazeuse de manière à suivre la conversion du propyne et du propadiène ainsi que la teneur en propylène. Les résultats obtenus sont présentés dans le tableau suivant :

| **Temps (heures)** | **Propylène (%poids)** | **Propyne (%poids)** | **Propadiène (%poids)** |
|---|---|---|---|
| 50 | 94,87 | - | 0,071 |
| 100 | 94,92 | - | 0,069 |
| 150 | 95,13 | - | 0,080 |
| 200 | 94,78 | - | 0,052 |
| 300 | 94,90 | - | 0,065 |

Si l'on calcule les teneurs moyennes en propadiène et en propylène pendant l'opération, on peut calculer la conversion moyenne en propyne et en propadiène ainsi que le gain moyen en propylène. On trouve dans cet exemple une conversion de 98 % et un rendement propylène exprimé par le rapport de la teneur en propylène du produit sur la teneur en propylène de la charge de 103 %.

### EXEMPLE 5 (selon l'invention)

Dans cet exemple on met en oeuvre la même réaction dans les mêmes conditions que dans l'exemple 4, mais en présence du catalyseur C de l'exemple 3. Les résultats d'analyse obtenus sont présentés dans le tableau suivant :

| **Temps (heures)** | **Propylène (%poids)** | **Propyne (%poids)** | **Propadiène (%poids)** |
|---|---|---|---|
| 50 | 95,41 | - | 0,0072 |
| 100 | 95,43 | - | 0,0089 |
| 150 | 95,39 | - | 0,0070 |
| 200 | 95,05 | - | 0,0090 |
| 300 | 95,56 | - | 0,0060 |

On trouve dans cet exemple une conversion de 99,78 % et un rendement propylène exprimé par le rapport de la teneur en propylène du produit sur la teneur en propylène de la charge de 103,5 %.

### EXEMPLE 6

Dans cet exemple on met en oeuvre la même réaction dans les mêmes conditions que dans l'exemple 1, mais cette fois on utilise successivement différents catalyseurs contenant 0,3 % poids de palladium et une teneur variable en gallium. Le support utilisé est identique à celui du catalyseur A monométallique de l'exemple 1. Différents lots de ce support sont imprégnés à sec par du nitrate de palladium en utilisant la même méthode que celle décrite dans l'exemple 1 pour le catalyseur A. On dépose ensuite le gallium par imprégnation de solutions de nitrate de gallium de concentrations variables. Après imprégnation, les échantillons sont séchés à une température de 120°C pendant 2 heures, puis calcinés sous débit d'air à une température de 450°C pendant 2 heures. Avant test, on réduit les catalyseurs sous débit d'hydrogène à une température de 150°C pendant 2 heures.

Le tableau suivant présente la composition du produit après 8 minutes de réaction pour chacun des catalyseurs repérés par leur teneur en gallium ainsi que pour le catalyseur A monométallique de l'exemple 1.

| **Teneur en Ga (% poids)** | **Butadiène (% poids)** | **Butènes (%poids)** | **Butane (%poids)** |
|---|---|---|---|
| 0 | 9 | 90,7 | 0,3 |
| 0,08 | 7,57 | 92,14 | 0,29 |
| 0,23 | 5,95 | 93,78 | 0,27 |
| 0,41 | 8,5 | 91,22 | 0,28 |
| 0,80 | 8,6 | 91,1 | 0,3 |
| 1,22 | 10,2 | 89,49 | 0,31 |

On remarque que les échantillons ayant une teneur en gallium comprise entre 0,08 et 0,80 % poids présentent une activité supérieure à celle du catalyseur monométallique de l'exemple 1 puisque au bout du même temps de réaction (8 minutes) la teneur en butadiène du produit est inférieure. De plus, on note que ces catalyseurs plus actifs que le monométallique sont aussi plus sélectifs vis à vis de l'hydrogénation consécutive des butènes. En effet, bien que la conversion du butadiène soit plus importante, on voit que la teneur en butènes est plus élevée et que la formation de butane est diminuée.

## Revendications

1. Procédé d'hydrogénation sélective d'impuretés dioléfiniques et/ou acétyléniques dans des charges contenant des monooléfines, consistant à mettre en contact la charge et l'hydrogène avec un catalyseur supporté, à une température comprise entre 0 et 200 °C et une pression comprise entre 20 et 300 bars, ledit procédé étant caractérisé en ce qu'il opère en l'absence de monoxyde de carbone et que le catalyseur renferme par rapport à sa masse totale :
a) 0,1 à 10 % d'au moins un métal du groupe VIII choisi dans le groupe constitué par le nickel, le palladium, le platine, le rhodium et le ruthénium ;
b) 0,01 à 10 % d'au moins un élément additionnel métallique du groupe III-A choisi dans le groupe constitué par le gallium et l'indium.

2. Procédé selon la revendication 1, caractérisé en ce que le métal du groupe VIII est choisi dans le groupe constitué par le palladium, le platine et le nickel.

3. Procédé selon l'une des revendications 1 ou 2 caractérisé en ce que le support est choisi dans le groupe constitué par une silice, une alumine, une silice-alumine un aluminate ou un aluminate mixte de métal alcalin, de métal alcalino terreux, du zinc et du cadmium.

4. Procédé selon l'un des revendications 1 à 3 caractérisé en ce que la concentration en métal du groupe VIII est comprise entre 0,2 et 5 % en poids.

5. Procédé selon l'une des revendications 1 à 4, caractérisé en ce que la concentration en métal du groupe III-A est comprise entre 0,1 et 5 % en poids.

6. Procédé selon l'une des revendications 1 à 5 caractérisé en ce que le rapport molaire élément métallique additionnel du groupe III sur métal du groupe VIII est compris entre 0,2 et 5.

7. Procédé selon l'une des revendications 1 à 6 caractérisé en ce que ledit rapport molaire est compris entre 0,3 et 2.

8. Procédé selon l'une des revendications 1 à 7, caractérisé en ce que le catalyseur est formé par dépôt du métal du group III-A suivi de dépôt de métal du groupe VIII.

9. Procédé selon la revendication 8, caractérisé en ce que le support imprégné du métal du groupe III-A est soumis à une réduction avant dépôt du métal du groupe VIII.

10. Procédé selon l'une des revendications précédentes, caractérisé en ce que le catalyseur est activé avant d'être mis au contact de la charge.

## Patentansprüche

1. Verfahren zur selektiven Hydrierung von diolefinischen und/oder acetylenischen Verunreinigungen in Chargen, welche Monoolefine enthalten, das darin besteht, die Charge und Wasserstoff mit einem Katalysator auf einem Träger bei einer Temperatur, die zwischen 0 und 200°C enthalten ist und einem Druck, der zwischen 20 und 300 bar enthalten ist, umzusetzen, wobei das Verfahren dadurch gekennzeichnet ist, daß es in Abwesenheit von Kohlenmonoxyd arbeitet und daß der Katalysator bezogen auf seine Gesamtmasse
a) 0,1 bis 10 % wenigstens eines Metalles der Gruppe VIII, ausgewählt aus der Gruppe, welche durch Nickel, Palladium, Platin, Rhodium und Ruthenium gebildet ist,
b) 0,01 bis 10 % eines zusätzlichen metallischen Elementes aus der Gruppe IIIA, ausgewählt aus der Gruppe, welche durch Gallium und Indium gebildet ist,
umfaßt.

2. Verfahren nach Anspruch 1, dadurch gekennzeichnet, daß das Metall der Gruppe VIII aus der Gruppe ausgewählt wird, welche durch Palladium, Platin und Nickel gebildet ist.

3. Verfahren nach einem der Ansprüche 1 oder 2, dadurch gekennzeichnet, daß der Träger aus der Gruppe ausgewählt wird, welche durch ein Siliziumdioxid, ein Aluminiumoxid, ein Siliziumdioxid-Aluminiumoxid, ein Aluminat oder ein gemischtes Aluminat von Alkalimetall, Erdalkalimetall, Zink und Cadmium gebildet ist.

4. Verfahren nach einem der Ansprüche 1 bis 3, dadurch gekennzeichnet, daß die Konzentration an Metall der Gruppe VIII zwischen 0,2 und 5 Gew.% enthalten ist.

5. Verfahren nach einem der Ansprüche 1 bis 4, dadurch gekennzeichnet, daß die Konzentration an Metall der Gruppe IIIA zwischen 0,1 und 5 Gew.% enthalten ist.

6. Verfahren nach einem der Ansprüche 1 bis 5, dadurch gekennzeichnet, daß das molare Verhältnis von zusätzlichem metallischem Element der Gruppe III zu Metall der Gruppe VIII zwischen 0,2 und 5 enthalten ist.

7. Verfahren nach einem der Ansprüche 1 bis 6, dadurch gekennzeichnet, daß das molare Verhältnis zwischen 0,3 und 2 enthalten ist.

8. Verfahren nach einem der Ansprüche 1 bis 7, dadurch gekennzeichnet, daß der Katalysator durch Abscheidung des Metalles der Gruppe IIIA nach Abscheidung des Metalles der Gruppe VIII gebildet wird.

9. Verfahren nach Anspruch 8, dadurch gekennzeichnet, daß der mit dem Metall der Gruppe IIIA imprägnierte Träger vor der Abscheidung des Metalles der Gruppe VIII einer Reduktion unterworfen wird.

10. Verfahren nach einem der vorhergehenden Ansprüche, dadurch gekennzeichnet, daß der Katalysator bevor er mit der Charge in Kontakt gebracht wird, aktiviert wird.

## Claims

1. Process for the selective hydrogenation of diolefin and/or acetylene impurities in charges containing nono-olefins, consisting of contacting the charge and the hydrogen with supported a catalyst at a temperature between 0 and 200°C and a pressure between 20 and 300 bars, said process being characterized in that it operates in the absence of carbon monoxide, and in that, based on its total weight, the catalyst contains :
a) 0.1 to 10% of at least one group VIII metal chosen from within the group constituted by nickel, palladium, platinum, rhodium and ruthenium,
b) 0.01 to 10% of at least one additional metallic element of group IIIA chosen from within the group constituted by gallium and indium.

2. Process according to claim 1, characterized in that the group VIII metal is chosen from among palladium, platinum and nickel.

3. Process according to either of the claims 1 and 2, characterized in that the support is chosen from within the group constituted by a silica, an alumina, a silica-alumina, an aluminate or a mixed alkali metal, alkaline earth metal, zinc or cadmium aluminate.

4. Process according to any one of the claims 1 to 3, characterized in that the group VIII metal concentration is between 0.2 and 5% by weight.

5. Process according to any one of the claims 1 to 4, characterized in that the group IIIA metal concentration is between 0.1 and 5% by weight.

6. Process according to any one of the claims 1 to 5, characterized in that the molar ratio of the additional group III metallic element to group VIII metal is between 0.2 and 5.

7. Process according to any one of the claims 1 to 6, characterized in that the molar ratio is between 0.3 and 2.

8. Process according to any one of the claims 1 to 7, characterized in that the catalyst is formed by the deposition of the group IIIA metal, followed by the deposition of the group VIII metal.

9. Process according to claim 8, characterized in that the support impregnated by the group IIIA metal undergoes a reduction prior to the deposition of the group VIII metal.

10. Process according to any one of the preceding claims, characterized in that the catalyst is activated before being contacted with the charge.
